# EUROPÄISCHE PATENTANMELDUNG

(11) **EP 1 184 043 A1**
(43) Veröffentlichungstag der Anmeldung: **06.03.2002**
(21) Anmeldenummer: 00118598.2
(22) Anmeldetag: 28.08.2000
(51) Int. Cl.: A61M 1/00

(54) **Kleinformatige Absaugpumpe**

(71) Anmelder: Medela AG, 6340 Baar (CH)
(72) Erfinder: Greter, Andy, 6312 Steinhausen (CH)
(74) Vertreter: Troesch Scheidegger Werner AG

(57) **Zusammenfassung**

Die kleinformatige Drainagepumpe, insbesondere Wunddrainagepumpe, umfasst einen Auffangbehälter (1) für abzusaugendes Material mit einem Deckel (2), wobei alle für den Betrieb erforderlichen Bestandteile (4 - 10) der Pumpe im Behälterdeckel (2) integriert sind.

## Beschreibung

Die vorliegende Erfindung betrifft eine kleinformatige Drainagepumpe, insbesondere für Wund-Drainage, mit einem Auffangbehälter mit Deckel für abzusaugendes Material, sowie funktionellen Bestandteilen, wie Vakuumpumpe mit Antrieb, Filter und Ueberwachungseinrichtungen für den Betrieb.

Bei der Wunddrainage wird üblicherweise eine (Sekret-) Absaugpumpe eingesetzt, deren Absaugleitung in Form eines Schlauches zu den Wundabdeckungen geführt wird. Die Absaugpumpe mit Auffangbehälter und Antriebseinheit (zur Erzeugung des gewünschten Vakuums) nimmt dabei relativ viel Platz ein und ist als stationäre Anlage zu betrachten.

Aufgabe der vorliegenden Erfindung war es, eine kleinformatige Drainagepumpe (Mini-Drainagepumpe) zu schaffen, welche auf möglichst kleinem Raum alle Bestandteile vereinigt und welche sich dadurch nicht ausschliesslich für stationären Einsatz eignet.

Um eine Pumpe mit im Vergleich zum Stand der Technik stark reduziertem Platzbedarf zu schaffen, wurde die Aufgabe bei einer Pumpe der eingangs definierten Art erfindungsgemäss dadurch gelöst, dass alle für den Betrieb erforderlichen Bestandteile, die sogenannten funktionellen Bestandteile, im Behälterdeckel integriert sind.

Besonders bevorzugte Ausführungsformen des Erfindungsgegenstandes sind in den abhängigen Ansprüchen definiert.

Die Erfindung wird nachstehend anhand eines in der Zeichnung dargestellten Ausführungsbeispiels noch etwas näher erläutert.

Es zeigt:

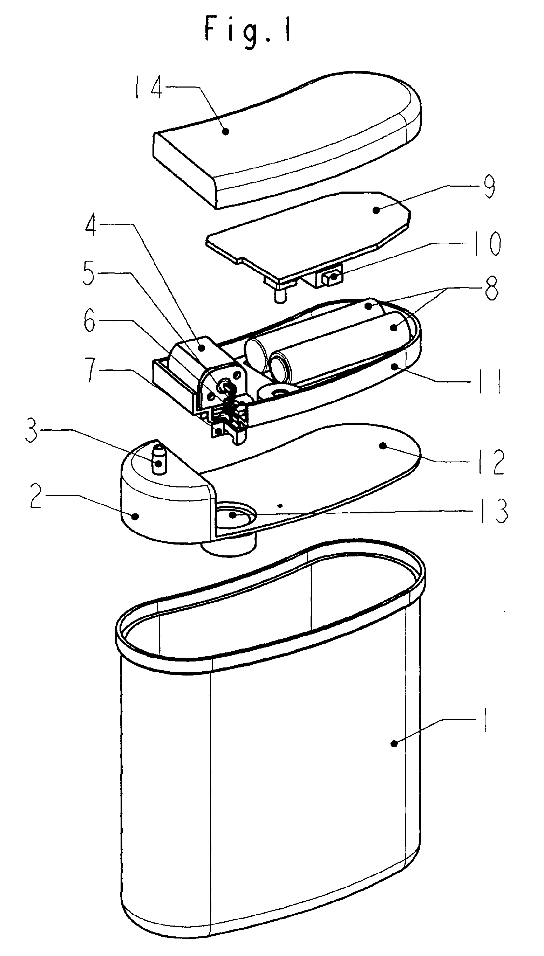

- Fig. 1: eine Absaugpumpe nach der Erfindung mit den verschiedenen Bestandteilen des Deckels vor dem Aufsetzen auf den Auffangbehälter (Explosionsdarstellung), und

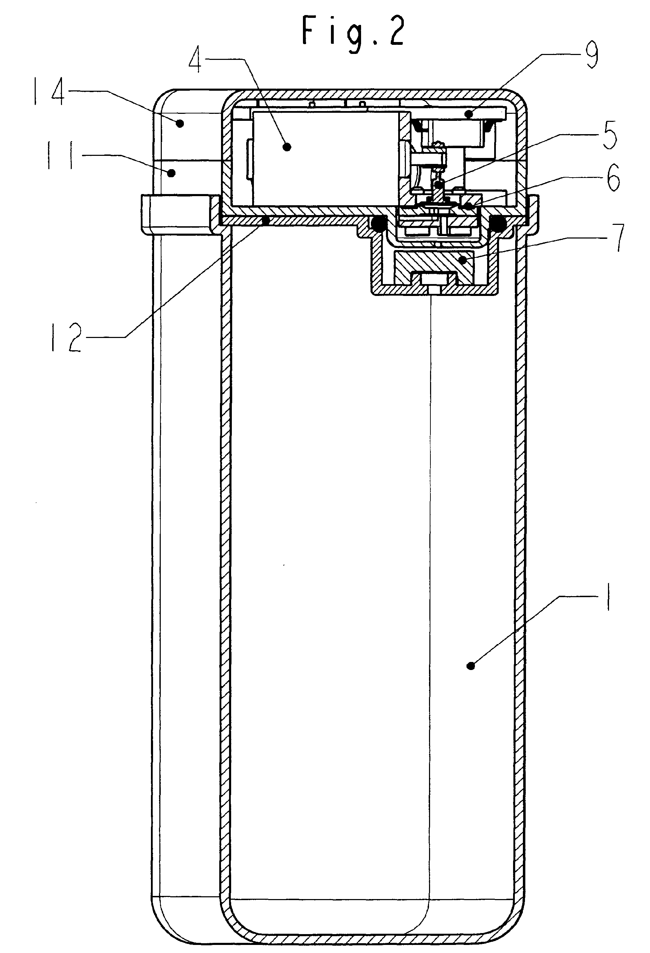

- Fig. 2: die fertig montierte Absaugpumpe nach Fig. 1 im Vertikalschnitt.

Die Zeichnung zeigt eine Absaugpumpe nach der Erfindung mit im Deckel angeordneten funktionellen Bestandteilen.

Die Absaugpumpe weist einen Auffangbehälter 1 mit dicht darauf aufsetzbarem Deckel 2 auf. Der Anschlussnippel 3 für einen Absaugschlauch (nicht dargestellt) erstreckt sich aus dem Deckel 2 nach oben.

Die für den Betrieb der Absaugpumpe erforderlichen Pumpenbestandteile, nämlich der Motor 4 mit Antriebspleuel 5 für eine Pumpenmembrane 6, ein der Membrane 6 vorgeschalteter hydrophober Filter 7, eine Batterie 8 und eine Steuer- und Ueberwachungselektronik 9, sowie ein Schalter 10 (Ein/Aus) sind als Einheit in einem Träger 11 integriert (z.B. eingegossen), wobei der Träger 11 mit den daran vereinten funktionellen Bestandteilen genau auf eine Grundplatte 12 des Deckels 2 aufsetzbar ist.

Die Membrane 6 der Pumpe wird über einen Durchlass 13 in der Grundplatte 12 des Deckels im Innern des Behälters ein Vakuum erzeugen und so das Absaugen von Wundsekret erlauben. Die vollkommen dicht in die obere Oeffnung des Auffangbehälters 1 lösbar einsetzbare Grundplatte 12 mit aufgesetztem Träger 11 kann schliesslich noch durch eine Abdeckung 14 abgedeckt werden.

## Patentansprüche

1. Kleinformatige Drainagepumpe, insbesondere für Wund-Drainage, mit einem Auffangbehälter (1) mit Deckel (2) für abzusaugendes Material, sowie funktionellen Bestandteilen, wie Vakuumpumpe mit Antrieb (4 - 8), Filter (7) und Ueberwachungseinrichtungen (9) für den Betrieb, **dadurch gekennzeichnet, dass** alle funktionellen Bestandteile (4 - 10) im Behälterdeckel (2) integriert sind.

2. Drainagepumpe nach Anspruch 1, **dadurch gekennzeichnet, dass** die funktionellen Bestandteile (4 - 10) als Baueinheit zusammengefasst lösbar in die obere Oeffnung des Auffangbehälters (1) eingelegt sind.

3. Drainagepumpe nach Anspruch 1 oder 2, **dadurch gekennzeichnet, dass** der Auffangbehälter (1) mit zugehörigem Deckel (2) im Horizontalschnitt nierenförmig ausgebildet ist, um optimal an menschlichen Körperteilen befestigt werden zu können.

4. Drainagepumpe nach einem der Ansprüche 1 bis 3, **dadurch gekennzeichnet, dass** am Behälter (1) Befestigungsmittel, wie z.B. Klettverschlussbänder vorgesehen sind.
